(19) Europäisches Patentamt
European Patent Office
Office européen des brevets

(11) EP 0 895 786 A1

(12) EUROPEAN PATENT APPLICATION

(43) Date of publication:
10.02.1999 Bulletin 1999/06

(51) Int Cl.6: A61M 1/18

(21) Application number: 98402005.7

(22) Date of filing: 06.08.1998

(84) Designated Contracting States:
AT BE CH CY DE DK ES FI FR GB GR IE IT LI LU MC NL PT SE
Designated Extension States:
AL LT LV MK RO SI

(30) Priority: 06.08.1997 JP 225875/97

(71) Applicant: TERUMO KABUSHIKI KAISHA
Tokyo (JP)

(72) Inventors:
- Naraoka, Hironobu
  Kitakatsusika-gun, Saitama-ken (JP)
- Takeuchi, Kazuhiko,
  c/o Terumo Kabushiki Kaisha
  Ashigarakami-gun, Kanagawa-ken (JP)

(74) Representative: Joly, Jean-Jacques et al
Cabinet Beau de Loménie
158, rue de l'Université
75340 Paris Cédex 07 (FR)

# (54) Oxygenator of hollow fiber membrane type

(57) The oxygenator 1 comprises a housing 3, a hollow fiber membrane bundle 2 comprising plurality of hollow fiber membranes for gas exchange and inserted in said housing, partitions 5, 6 for supporting opposite ends of said hollow fiber membrane bundle 2 to the housing 3, respectively, with said both ends of the hollow fiber membranes being open, a blood inlet 8 and blood outlets 9a and 9b all communicating with a blood chamber 7 formed in the housing 3, a gas inlet 11 and a gas outlet 12 both communicating with the interior of the hollow fiber membranes. The hollow fiber membrane bundle 2 has a substantially uniform thickness (x: mm). The following relationship is satisfied between the thickness (x) of the hollow fiber membrane bundle 2 and a loading density (y: %) of the hollow fiber membranes to an annular space (z) formed between the outer surface (cylindrical outer surface) and the inner surface (cylindrical inner surface) of the hollow fiber membrane bundle 2:

$$y \leq -0.75x + 84 \quad \text{(Equation 1)}$$

$$y \geq -0.85x + 73 \quad \text{(Equation 2)}$$

$$5 \leq x \leq 31 \quad \text{(Equation 3)}$$

F I G. 1

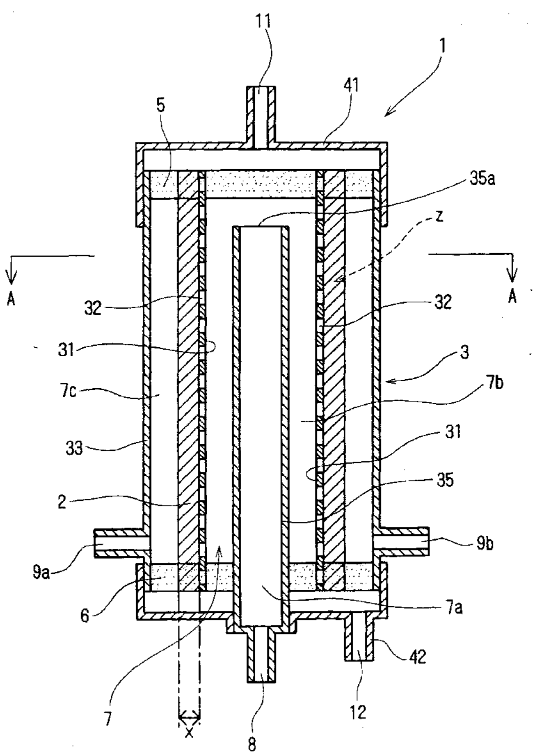


EP 0 895 786 A1

## Description

BACKGROUND OF THE INVENTION

The present invention relates to an oxygenator, of hollow fiber membrane type, for removing carbon dioxide from blood and oxygenate the blood in an extracorporeal blood circulation .

A membrane type oxygenator is now in wide use. The membrane type oxygenator is composed mainly of hollow fiber membranes through which oxygen and carbon dioxide in the blood are exchanged with each other.

As the method of introducing the blood into the membrane type oxygenator, there are internal circulation type and an external circulation type. In the former type, the blood is introduced into the inner side of the hollow fiber membranes, whereas the gas is flowed to the outer side thereof. In the latter type, the blood is introduced into the outer side of the hollow fiber membranes, whereas the gas is flowed to the inner side thereof. It is known that in circulating the blood, the degree of pressure loss in the former type is larger than that in the latter type.

The pressure loss of the membrane type oxygenator is preferably small to circulate the blood extracorporeally by a centrifugal pump or a pulsation flow pump, the use of which has spread in recent years. Because it is possible to form a blood flowing duct freely in the membrane type oxygenator adopting the external circulation type, the blood is likely to flow nonuniformly in the membrane type oxygenator. Consequently, in the membrane type oxygenator adopting the external circulation type, when the loading density of the hollow fiber membranes to an annular space formed between the outer surface and the inner surface of a bundle consisting of the hollow fiber membranes is increased to improve gas exchange efficiency and flow the blood uniformly, the degree of the pressure loss becomes high.

Therefore, it is an object of the present invention to provide an oxygenator of external circulation type allowing a low degree of pressure loss and a high degree of gas exchange efficiency to be obtained and allowing blood to flow uniformly therein.

SUMMARY OF THE INVENTION

In order to achieve the object, there is provided an oxygenator of hollow fiber membrane type comprises a housing, a hollow fiber membrane bundle comprising plurality of hollow fiber membranes for gas exchange and inserted in said housing, partitions for supporting opposite ends of said hollow fiber membrane bundle to said housing, respectively, with said both ends of said hollow fiber membranes being open, a blood inlet and a blood outlet both communicating with a blood chamber formed in said housing, a gas inlet and a gas outlet both communicating with said interior of said hollow fiber membranes, wherein said hollow fiber membrane bundle has a substantially uniform thickness (x); and the following relationship is satisfied between said thickness (x: mm) of said hollow fiber membrane bundle and a loading density (y: %) of said hollow fiber membranes to an annular space (z) formed between said outer surface and said inner surface of said hollow fiber membrane bundle:

$$y \leq -0.75x + 84 \qquad \text{(Equation 1)}$$

$$y \geq -0.85x + 73 \qquad \text{(Equation 2)}$$

$$5 \leq x \leq 31 \qquad \text{(Equation 3)}$$

BRIEF DESCRIPTION OF THE DRAWINGS

The foregoing and further objects, features and advantages of the present invention will become apparent from the following description of preferred embodiments with reference to the accompanying drawings, wherein like numerals are used to represent like elements and wherein:

Fig. 1 is a sectional view showing an oxygenator of hollow fiber membrane type according to an embodiment of the present invention.

Fig. 2 is a sectional view taken along a line A-A of Fig. 1.

Fig. 3 is a front view showing an example of an inner tubular member to be used for the oxygenator of hollow fiber membrane type of the present invention.

Fig. 4 is a longitudinal view showing the inner tubular member shown in Fig. 3.

Fig. 5 is a sectional view taken along a line B-B of Fig. 3.

Fig. 6 is a graph for describing the condition which is satisfied by the oxygenator of hollow fiber membrane type of the present invention.

Fig. 7 is a graph for describing a preferable condition which is satisfied by the oxygenator of hollow fiber membrane type of the present invention.

Fig. 8 is a graph for describing a preferable condition which is satisfied by the oxygenator of hollow fiber membrane type of the present invention.

Fig. 9 is a graph for describing a preferable condition which is satisfied by the oxygenator of hollow fiber membrane type of the present invention.

DETAILED DESCRIPTION OF THE PREFERRED EMBODIMENTS

The oxygenator of hollow fiber membrane type (hereinafter referred to merely as oxygenator) of the present invention will be described below with reference to drawings.

The oxygenator 1 of the present invention comprises a housing 3, a hollow fiber membrane bundle 2 comprising plurality of hollow fiber membranes for gas exchange and inserted in said housing 3, partitions 5, 6 for supporting opposite ends of said hollow fiber membrane bundle 2 to the housing 3, respectively, with said both ends of the hollow fiber membranes being open, a blood inlet 8 and blood outlets 9a and 9b all communicating with a blood chamber 7 formed in the housing 3, a gas inlet 11 and a gas outlet 12 both communicating with the interior of the hollow fiber membranes. The hollow fiber membrane bundle 2 has a substantially uniform thickness (x: mm). The following relationship is satisfied between the thickness (x) of the hollow fiber membrane bundle 2 and a loading density (y: %) of the hollow fiber membranes to an annular space (z) formed between the outer surface (cylindrical outer surface) and the inner surface (cylindrical inner surface) of the hollow fiber membrane bundle 2:

$$y \leq -0.75x + 84 \qquad \text{(Equation 1)}$$

$$y \geq -0.85x + 73 \qquad \text{(Equation 2)}$$

$$5 \leq x \leq 31 \qquad \text{(Equation 3)}$$

In the hollow fiber blood oxygenator 1 of the embodiment shown in Figs. 1 and 2, the housing 3 comprises an inner tubular member 31 having a large number of blood flowing apertures 32 provided to a side wall thereof and an outer tubular member 33 receiving the inner tubular member 31. The hollow fiber membrane bundle 2 is located between the inner tubular member 31 and the outer tubular member 33. The oxygenator 1 has an interior ( blood inlet portion 7a) of the inner tubular member 31 communicating with one of the blood inlet 8 or the blood outlets 9a and. Otherwise, the oxygenator 1 has an interior ( second blood chamber 7c) of the outer tubular member 33 communicating with one of the blood inlet 8 of the blood outlets 9a and. The outer side blood chamber is a tubular space formed between the outer surface of the hollow fiber membrane bundle 2 and an inner surface of the outer tubular member 33.

More specifically, the housing 3 of the oxygenator 1 of the embodiment comprises the outer tubular member 33, the inner tubular member 31, and a central tubular member 35 receiving the inner tubular member 31 and open at the leading (upper) end thereof. The central tubular member 35 has the blood inlet 8 formed at one (lower) end thereof. The blood outlets 9a and 9b project outward from the peripheral surface of the outer tubular member 33. Only one blood outlet 9a or 9b may be formed on the outer tubular member 33.

The hollow fiber membrane bundle 2 is wound on the peripheral surface of the inner tubular member 31. That is, the inner tubular member 31 serves as the core of the hollow fiber membrane bundle 2. The other end (upper end) 35a of the central tubular member 35 accommodated inside the inner tubular member 31 is open in the neighborhood of the first partition 5.

The central tubular member 35, the inner tubular member 31 around which the hollow fiber membrane bundle 2 is wound, and the outer tubular member 33 are substantially concentric or coaxial with one another. The first partition 5 serves as the means for securing thereto the upper end of the inner tubular member 31 and that of the outer tubular member 33 to keep the concentric relationship between the inner tubular member 31 and the outer tubular member 33 and keep liquid-tight both the space formed between the central tubular member 35 and the inner tubular member 31 and the space formed between the outer tubular member 33 and the outer surface of the hollow fiber membrane bundle 2 so that the spaces do not communicate with the outside.

The second partition 6 serves as the means for securing or supporting thereto the lower end of the inner tubular member 31 and that of the outer tubular member 33 both positioned a little upward from the blood inlet 8 of the central tubular member 35 to keep the concentric relationship between the inner tubular member 31 ad the outer tubular member 33 and keep liquid-tight both the space formed between the central tubular member 35 and the inner tubular member 31 and the space formed between the outer tubular member 33 and the outer surface of the hollow fiber membrane bundle 2 so that the spaces do not communicate with the outside. The first and second partitions 5 and 6 are formed of potting agent such as polyurethane and silicon rubber.

In the oxygenator 1 of the embodiment has a blood inlet portion 7a formed inside the central tubular member 35, a first blood chamber 7b formed between the central tubular member 35 and the inner tubular member 31, and a second blood chamber 7c formed between the hollow fiber membrane bundle 2 and the outer tubular member 33.

Blood which has entered the oxygenator 1 from the blood inlet 8 flows to the blood inlet portion 7a, moving upward inside the central tubular member 35 (blood inlet portion 7a). The blood which has flowed out from the upper free end 35a of the central tubular member 35 flows into the first blood chamber 7b, thus passing through the blood flowing apertures 32 formed on the inner tubular member 31 and contacting the hollow fiber membranes through which gas exchange is performed. Then, the blood flows into the second blood chamber 7c, thus flowing out from the oxygenator 1 through the blood outlets 9a and 9b.

A gas introducing member 41 provided with the gas inlet 11 is fixed to one end (upper end) of the outer tubular member 33. Similarly, a gas exhaust member 42 provided with the gas outlet 12 is fixed to the other end (lower end) of the outer tubular member 33. The blood inlet 8 of the central tubular member 35 penetrates through the gas exhaust member 42, thus projecting downward to the outside.

The outer tubular member 33 can be formed of a member annular, polygonal or elliptic in section. It is preferable to use a cylindrical member. The inner diameter of the outer tubular member 33 is preferably in the range of 32mm to 164mm. The effective length (length excluding the portion surrounded with the partitions 5 and 6) of the outer tubular member 33 is preferably in the range of 10mm to 730mm.

Similarly, the inner tubular member 31 can be formed of a member annular, polygonal or elliptic in section. It is preferable to use a cylindrical member. The outer diameter of the inner tubular member 31 is preferably in the range of 20mm to 100mm. The effective length (length excluding the portion surrounded with the partitions 5 and 6) of the inner tubular member 31 is preferably in the range of 10mm to 730mm. The inner tubular member 31 is provided with a large number of the blood flowing apertures 32 formed thereon. It is preferable that the total area of the blood flowing apertures 32 is as large as possible so long as they allow the inner tubular member 31 to have a required degree of strength. In order to satisfy the condition, it is preferable that a plurality (4 pieces to 24 pieces) of the blood flowing apertures 32 are formed on the peripheral surface of the inner tubular member 31 such that they are spaced at regular central angles in the circumferential direction of the inner tubular member 31 and at regular intervals in the axial direction thereof, as shown in Fig.3 which is a front view showing the oxygenator 1, Fig. 4 which is a vertical sectional view of Fig. 3, and Fig. 5 which is a sectional view taken along the line B-B of Fig. 3. In the embodiment, eight blood flowing apertures 32 are formed thereon circumferentially and axially. The blood flowing apertures 32 may be circular, polygonal or elliptic, but they are preferably capsule-shaped.

The central tubular member 35 can be formed of a material annular, polygonal or elliptic in section. It is preferable to use an annular material. The distance between the upper end 35a of the central tubular member 35 and the partition 5 is preferably in the range of 20mm to 50mm. The inner diameter of the central tubular member 35 is preferably in the range of 10mm to 30mm.

The hollow fiber membrane bundle 2 is wound around the peripheral surface of the inner tubular member 31. The following relationship is satisfied between the thickness (x) of the hollow fiber membrane bundle 2 and a loading density (y: %) of the hollow fiber membranes to an annular space (z) formed between the outer surface (cylindrical outer surface) and the inner surface (cylindrical inner surface) of the hollow fiber membrane bundle 2:

$$y \leq -0.75x + 84 \qquad \text{(Equation 1)}$$

$$y \geq -0.85x + 73 \qquad \text{(Equation 2)}$$

$$5 \leq x \leq 31 \qquad \text{(Equation 3)}$$

The annular space (z) is the space defined by the peripheral surface of the hollow fiber membrane bundle 2, the inner peripheral surface thereof, the upper end thereof, and the lower end thereof, excluding the portion thereof embedded in the partitions 5 and 6. In other words, the annular space (z) denotes the volume of the hollow fiber membrane bundle 2.

$$y \text{ ( loading density or winding density)} = (AN/B) \times 100(\%)$$

A: cross sectional area defined by the outer surface of the one hollow fiber membrane
N: number of hollow fiber membranes
A: cross sectional area defined between the outer surface of the hollow fiber membrane bundle and the inner surface of the hollow fiber membrane bundle ( cross sectional area of the annular space (z) )

Because the hollow fiber membrane bundle 2 satisfies the above equations, the oxygenator 1 of the present invention has an improved pressure loss, gas exchange efficiency, and uniform flow of blood in the hollow fiber membrane bundle. Examinations were made on oxygenators satisfying the condition that oxygen permeation amount (index of gas exchange percentage) was 360ml/minute or more and pressure loss was 80mmHg or less or 75mmHg or less. As a result, it was possible to obtain those which satisfied the condition of each of the above equations (1) through (3) .
The range satisfying equations (1) through (3) is indicated by the oblique line region of the graph shown in Fig. 6.
It is preferable for the hollow fiber membrane bundle 2 to satisfy the following condition:

$$50 \leq y \leq 75 \qquad \text{(Equation 4)}$$

Thus, the range satisfying equations (1), (2), (3), and (4) is indicated by the oblique line region of the graph shown in Fig. 7.
It is preferable for the hollow fiber membrane bundle 2 to satisfy the following-condition:

$$y \leq -0.75x + 82 \qquad \text{(Equation 5)}$$

$$y \geq -0.85x + 74 \qquad \text{(Equation 6)}$$

$$5 \leq x \leq 31 \qquad \text{(Equation 3)}$$

$$50 \leq y \leq 75 \qquad \text{(Equation 4)}$$

The hollow fiber membrane bundle 2 satisfying the above conditions has a small pressure loss.
Thus, the range satisfying equations (5), (6), (3), and (4) is indicated by the oblique line region of the graph shown in Fig. 8.
It is preferable for the hollow fiber membrane bundle 2 to satisfy the following condition:

$$y \leq -0.75x + 82 \qquad \text{(Equation 5)}$$

$$y \geq -0.85x + 74 \qquad \text{(Equation 6)}$$

$$5 \leq x \leq 31 \qquad \text{(Equation 3)}$$

$$53 \leq y \leq 73 \qquad \text{(Equation 7)}$$

The hollow fiber membrane bundle 2 satisfying the above conditions has a small pressure loss.
Thus, the range satisfying equations (5), (6), (3), and (7) is indicated by the oblique line region of the graph shown in Fig. 9.
The outer diameter of the hollow fiber membrane bundle 2 is preferably in the range of 30mm to 162mm.
The hollow fiber membrane is used as a porous hollow fiber. The inner diameter of the porous fiber membrane composing the hollow fiber membrane bundle 2 is preferably in the range of 100μm to 1000μm. The thickness of the porous fiber membrane is favorably in the range of 5 - 200μm, and more favorably in the range of 10μm to 100μm. The open area ratio of the porous fiber membrane is favorably in the range of 20% to 80% and more favorably in the range of 30% to 60%. The diameter of the micro-pore of the porous fiber membrane is favorably in the range of 0.01μm to 5μm, and more favorably in the range of 0.01μm to- 1μm.

The porous fiber membrane can be formed of hydrophobic macromolecular materials such as polypropylene, polyethylene, polysulfone, polyacrylonitrile, polytetrafluoroethylene, and cellulose acetate. It is favorable to use polyolefin resin. It is more favorable to use polypropylene. Drawing method or solid phase/liquid phase separation method can be preferably used to form micro-pores on the material of the porous fiber membrane. The thickness of the hollow fiber membrane bundle 2 is preferably in the range of 10mm to 28mm ($10 \leq x \leq 28$mm).

The hollow fiber membrane bundle 2 satisfying the above-described condition is formed as follows: The hollow fiber membranes are wound around the inner tubular member 31 to form an hollow fiber membrane bobbin and to satisfy (Equation 1) , (Equation 2) and (Equation 3), utilizing the inner tubular member 31 as the core thereof. In other words, the loading density is a winding density.

Then, both ends of the formed hollow fiber membrane bobbin are fixed to the partitions 5 and 6, and are then cut off, together with the inner tubular member 31 serving as the core of the hollow fiber membrane bobbin. As a result, both ends of the hollow fiber membranes are open on the peripheral surface of the partitions 5 and 6.

To suppress nonuniform flow of blood (blood short passage), it is preferable to wind hollow fiber membranes one by one or a combination of a plurality thereof around the inner tubular member 31 in such a manner that they are substantially parallel with one another and spaced at substantially regular intervals. The interval between the hollow fiber membranes adjacent to each other is favorably set to 1/10 to 1/1 of the outer diameter of the hollow fiber membrane. More specifically, the interval between the adjacent fiber membranes is set to favorably 30μm to 200μm and more favorably 50μm to 180μm.

It is preferable to form the hollow fiber membrane bundle 2 by winding hollow fiber membranes one by one or a combination of a plurality thereof around the inner tubular member 31 in such a manner that they are substantially parallel with one another and spaced at substantially regular intervals. Moreover, when the hollow fiber membranes are wound around the inner tubular member 31, it is preferable to form the hollow fiber membrane bundle 2 by operating a rotary member for rotating the inner tubular member 31 and a winder for threading the hollow fiber membranes in such a manner that the rotary member and the winder satisfy the following equation:

traverse [mm/lot] x n (integer) = traverse swinging width x 2 ± (outer diameter of hollow fiber membrane + interval between adjacent hollow fiber membranes) x number of hollow fiber membranes wound around the inner tubular member 31. (Equation 8)

The above-described method of forming the hollow fiber membrane bundle 2 prevents blood from being nonuniformly flowing (blood short passage) therethrough to a great degree. The above-described integer (n) representing the relationship between the number of rotations of the winding rotary member and the number of reciprocations of the winder should be set to favorably 1 to 5 and more favorably to 2 to 4.

The oxygenator 1 described in the embodiment is so constructed that blood flows through the hollow fiber membrane bundle 2 from the inner side thereof to the outer side thereof, thus flowing out from the oxygenator 1. But the oxygenator 1 of the present invention is not limited to this type of oxygenator 1. For example, the oxygenator 1 is so constructed that blood flows through the hollow fiber membrane bundle 2 from the outer side thereof to the inner side thereof, thus flowing out from the oxygenator 1. In the oxygenator 1described in the embodiment, the inner tubular member 31 which is used as the core in forming the hollow fiber membrane bundle 2 is not removed from the oxygenator 1 to maintain the shape of the hollow fiber membrane bundle 2. But the oxygenator 1 is not necessarily provided with the inner tubular member 31. That is, the inner tubular member 31 may be removed from the oxygenator 1 after the hollow fiber membrane bundle 2 is formed by winding it around the inner tubular member 31.

EXAMPLE

The example of the oxygenator of the present invention and comparative example will be described below.

The inner tubular member 31 used had an outer diameter of 50mm and a length of 188mm. Four hollow fiber membranes made of porous polypropylene were wound around the inner tubular member 31 one upon another at regular intervals. Each of the hollow fiber membranes had an inner diameter of 195µm, an outer diameter of 295µm, and a porosity of about 35%. Then, a plurality of hollow fiber membranes was wound on the outermost hollow fiber membrane of the four hollow fiber membranes at the same interval set in the case of the four intervals to form an hollow fiber membrane bobbin.

The hollow fiber membrane bobbin was accommodated in the outer tubular member 33. Then, one end of the hollow fiber membrane bobbin was fixed with potting agent and then cut off. Then, the central tubular member 35 was inserted into the hollow fiber membrane bobbin. Thereafter, the other end thereof was fixed to partitions with potting agent. Then, the other end of the hollow fiber membrane bobbin was cut off without cutting the central tubular member 35 while the hollow fiber membrane bobbin was rotating on the central tubular member 35. Then, a gas introduction member and a gas discharging member were installed on the outer tubular member 33. In this manner, an oxygenator comprising an hollow fiber membrane bundle having the construction as shown in Fig. 1 was prepared.

Oxygenators of the example and those of the comparative example were prepared by altering the conditions shown in tables shown below. The area of each hollow fiber membrane was set to $2.5m^2$.

TABLE 1

| | Interval between | Fiber bundle | | Loading density | Satisfied equations |
|---|---|---|---|---|---|
| | fiber (µm) | Thickness (x) (mm) | Length (mm) | (y)(%) | |
| Embodiment 1 | 50 | 10 | 153 | 65 | 1,2,3,4 |
| Embodiment 2 | 50 | 10 | 149 | 67 | 1,2,3,4 |
| Embodiment 3 | 50 | 10 | 142 | 70 | 1,2,3,4 |
| Embodiment 4 | 50 | 10 | 136 | 73 | 1,2,3,4 |
| Embodiment 5 | 50 | 10 | 133 | 75 | 1,2,3,4 |
| Embodiment 6 | 50 | 10 | 129 | 77 | 1,2,3,4 |
| Comparative example 1 | 50 | 10 | 161 | 62 | 1,3 |
| Comparative example 2 | 50 | 10 | 128 | 80 | 2,3 |

TABLE 2

| | Interval between | Fiber bundle | | Loading density | Satisfied equations |
|---|---|---|---|---|---|
| | fiber (µm) | Thickness(x) (mm) | Length (mm) | (y)(%) | |
| Embodiment 7 | 80 | 16 | 91 | 62 | 1,2,3,4 |
| Embodiment 8 | 80 | 16 | 87 | 65 | 1,2,3,4 |
| Embodiment 9 | 80 | 16 | 82 | 69 | 1,2,3,4 |
| Embodiment 10 | 80 | 16 | 81 | 70 | 1,2,3,4 |
| Embodiment 11 | 80 | 16 | 79 | 72 | 1,2,3,4 |
| Comparative example 3 | 80 | 16 | 94 | 59 | 1,3 |
| Comparative example 4 | 80 | 16 | 75 | 75 | 2,3,4 |

TABLE 3

| | Interval between | Fiber bundle | | Loading density | Satisfied equations |
|---|---|---|---|---|---|
| | fiber(µm) | Thickness (x) (mm) | Length (mm) | (y)(%) | |
| Embodiment 12 | 100 | 17.5 | 84 | 60 | 1,2,3,4 |
| Embodiment 13 | 100 | 17.5 | 82 | 62 | 1,2,3,4 |
| Embodiment 14 | 100 | 17.5 | 78 | 65 | 1,2,3,4 |
| Embodiment 15 | 100 | 17.5 | 74 | 68 | 1,2,3,4 |
| Embodiment 16 | 100 | 17.5 | 72 | 70 | 1,2,3,4 |
| Comparative example 5 | 100 | 17.5 | 87 | 58 | 1,3,4 |
| Comparative example 6 | 100 | 17.5 | 70 | 72 | 2,3,4 |

TABLE 4

| | Interval between fiber (µm) | Fiber bundle Thickness (x) (mm) | Fiber bundle Length (mm) | Loading density (y)(%) | Satisfied equations |
|---|---|---|---|---|---|
| Embodiment 17 | 120 | 20 | 74 | 58 | 1,2,3,4 |
| Embodiment 18 | 120 | 20 | 71 | 60 | 1,2,3,4 |
| Embodiment 19 | 120 | 20 | 66 | 65 | 1,2,3,4 |
| Embodiment 20 | 120 | 20 | 65 | 66 | 1,2,3,4 |
| Embodiment 21 | 120 | 20 | 63 | 68 | 1,2,3,4 |
| Comparative example 7 | 120 | 20 | 76 | 55 | 1,3,4 |
| Comparative example 8 | 120 | 20 | 61 | 70 | 2,3,4 |

TABLE 5

| | Interval between fiber (µm) | Fiber bundle Thickness (x) (mm) | Fiber bundle Length (mm) | Loading density (y)(%) | Satisfied equations |
|---|---|---|---|---|---|
| Embodiment 22 | 150 | 26 | 57 | 53 | 1,2,3,4 |
| Embodiment 23 | 150 | 26 | 54 | 56 | 1,2,3,4 |
| Embodiment 24 | 150 | 26 | 52 | 58 | 1,2,3,4 |
| Embodiment 25 | 150 | 26 | 50 | 60 | 1,2,3,4 |
| Embodiment 26 | 150 | 26 | 48 | 63 | 1,2,3,4 |
| Comparative example 9 | 150 | 26 | 60 | 50 | 1,3,4 |
| Comparative example 10 | 150 | 26 | 46 | 65 | 2,3,4 |

TABLE 6

| | Interval between fiber (µm) | Fiber bundle Thickness (x) (mm) | Fiber bundle Length (mm) | Loading density (y)(%) | Satisfied equations |
|---|---|---|---|---|---|
| Embodiment 27 | 200 | 30.5 | 52 | 47 | 1,2,3,4 |
| Embodiment 28 | 200 | 30.5 | 49 | 50 | 1,2,3,4 |
| Embodiment 29 | 200 | 30.5 | 46 | 53 | 1,2,3,4 |
| Embodiment 30 | 200 | 30.5 | 44 | 55 | 1,2,3,4 |
| Embodiment 31 | 200 | 30.5 | 43 | 57 | 1,2,3,4 |
| Embodiment 32 | 200 | 30.5 | 41 | 60 | 1,2,3,4 |
| Comparative example 11 | 200 | 30.5 | 55 | 44 | 1,3,4 |
| Comparative example 12 | 200 | 30.5 | 39 | 63 | 2,3,4 |
| Comparative example 13 | 200 | 8 | 234 | 55 | 1,2,4 |
| Comparative example 14 | 2000 | 32 | 41 | 55 | 1,2,4 |

The rotary member for rotating the inner tubular member 31 and the winder for threading the hollow fiber membranes satisfied the equation (8). As the integer (n) indicating the relationship between the number of rotations of the rotary member and the number of reciprocations of the winder, 4 is selected in the embodiment.

Experiment

Using bovine blood, an experiment shown below was conducted on the oxygenators of the embodiments and those of the comparative example prepared in the above-described manner. The bovine blood used in the experiment and the comparative example was standard venous blood specified by AMMI (Association for the Advance of Medical Instrumentation). The anticoagulant-added blood was introduced into each oxygenator at a flow rate of 7L/minute. The blood was collected at a position proximate to the blood inlet of each oxygenator and the blood outlet thereof to measure the partial pressure of oxygen, the partial pressure of carbon dioxide, and pH by a blood/gas analyzing device. In this manner, the movement amount of oxygen and that of carbon dioxide were determined. A pressure loss at the blood flow rate of 7L/minute was measured. The result is as shown in the following tables.

TABLE 7

| | Oxygen movement amount (ml/min) | Pressure loss (mmHg) |
|---|---|---|
| Embodiment 1 | 382.3 | 321 |
| Embodiment 2 | 385.6 | 35.4 |
| Embodiment 3 | 389.4 | 38.5 |
| Embodiment 4 | 392.5 | 42.6 |
| Embodiment 5 | 395.4 | 48.0 |
| Embodiment 6 | 400.1 | 52.3 |
| Comparative example 1 | 348.2 | 28.5 |
| Comparative example 2 | 420.5 | 80.5 |

TABLE 8

| | Oxygen movement amount (ml/min) | Pressure loss (mmHg) |
|---|---|---|
| Embodiment 7 | 378.2 | 38.5 |
| Embodiment 8 | 380.5 | 42.1 |
| Embodiment 9 | 384.6 | 46.3 |
| Embodiment 10 | 388.9 | 51.2 |
| Embodiment 11 | 392.5 | 57.4 |
| Comparative example 3 | 349.6 | 31.5 |
| Comparative example 4 | 408.3 | 81.3 |

TABLE 9

| | Oxygen movement amount (ml/min) | Pressure loss (mmHg) |
|---|---|---|
| Embodiment 12 | 374.3 | 39.5 |
| Embodiment 13 | 386.1 | 44.2 |
| Embodiment 14 | 388.4 | 50.0 |
| Embodiment 15 | 391.5 | 53.8 |
| Embodiment 16 | 395.2 | 59.2 |
| Comparative example 5 | 348.6 | 33.4 |
| Comparative example 6 | 420.5 | 83.4 |

TABLE 10

| | Oxygen movement amount (ml/min) | Pressure loss (mmHg) |
|---|---|---|
| Embodiment 17 | 383.1 | 45.3 |
| Embodiment 18 | 385.8 | 50.1 |
| Embodiment 19 | 389.2 | 55.6 |
| Embodiment 20 | 392.5 | 60.2 |
| Embodiment 21 | 396.0 | 65.8 |
| Comparative example 7 | 347.6 | 38.7 |
| Comparative example 8 | 415.1 | 85.1 |

TABLE 11

| | Oxygen movement amount (ml/min) | Pressure loss (mmHg) |
|---|---|---|
| Embodiment 22 | 383.5 | 49.5 |
| Embodiment 23 | 387.4 | 53.6 |
| Embodiment 24 | 391.6 | 58.4 |
| Embodiment 25 | 394.9 | 62.8 |
| Embodiment 26 | 398.2 | 67.1 |
| Comparative example 9 | 349.0 | 39.1 |
| Comparative example 10 | 421.5 | 86.1 |

TABLE 12

| | Oxygen movement amount (ml/min) | Pressure loss (mmHg) |
|---|---|---|
| Embodiment 27 | 379.5 | 50.6 |
| Embodiment 28 | 382.8 | 53.1 |
| Embodiment 29 | 386.1 | 56.2 |
| Embodiment 30 | 390.4 | 60.0 |
| Embodiment 31 | 393.8 | 65.2 |
| Embodiment 32 | 398.0 | 69.8 |
| Comparative example 11 | 346.8 | 40.2 |
| Comparative example 12 | 430.4 | 81.6 |
| Comparative example 13 | 289.5 | 10.4 |
| Comparative example 14 | 434.1 | 83.7 |

The oxygenator of the present invention has a function allowing a low degree of pressure loss and a high degree of gas exchange efficiency to be obtained and blood to flow uniformly therein.

While the present invention has been described with reference to what are presently considered to be preferred embodiments thereof, it is to be understood that the invention is not limited to the disclosed embodiments or constructions. To the contrary, the invention is intended to cover various modifications and equivalent arrangements included within the spirit and scope of the appended claims.

**Claims**

**1.** An oxygenator of hollow fiber membrane type comprising

a housing,
a hollow fiber membrane bundle comprising plurality of hollow fiber membranes for gas exchange and inserted in said housing,
partitions for supporting opposite ends of said hollow fiber membrane bundle to said housing, respectively, with said both ends of said hollow fiber membranes being open,
a blood inlet and blood outlet both communicating with a blood chamber formed in said housing,
a gas inlet and a gas outlet both communicating with said interior of said hollow fiber membranes,
wherein said hollow fiber membrane bundle has a substantially uniform thickness (x); and the following relationship is satisfied between said thickness (x: mm) of said hollow fiber membrane bundle and a loading density (y: %) of said hollow fiber membranes to an annular space (z) formed between said outer surface and said inner surface of said hollow fiber membrane bundle:

$$y \leq -0.75x + 84 \qquad \text{(Equation 1)}$$

$$y \geq -0.85x + 73 \qquad \text{(Equation 2)}$$

$$5 \leq x \leq 31 \quad \text{(Equation 3)}$$

2. The oxygenator of hollow fiber membrane type according to claim 1, wherein the following relationship is satisfied between said thickness (x: mm) of said hollow fiber membrane bundle and said loading density (y: %) of said hollow fiber membranes to said annular space (z) formed between said outer surface and said inner surface of said hollow fiber membrane bundle:

$$y \leq -0.75x + 84 \quad \text{(Equation 1)}$$

$$y \geq -0.85x + 73 \quad \text{(Equation 2)}$$

$$5 \leq x \leq 31 \quad \text{(Equation 3)}$$

$$50 \leq y \leq 75 \quad \text{(Equation 4)}$$

3. The oxygenator of hollow fiber membrane type according to claim 1, wherein the following relationship is satisfied between said thickness (x: mm) of said hollow fiber membrane bundle and said loading density (y: %) of said hollow fiber membranes to said annular space (z) formed between said outer surface and said inner surface of said hollow fiber membrane bundle:

$$y \leq -0.75x + 82 \quad \text{(Equation 5)}$$

$$y \geq -0.85x + 74 \quad \text{(Equation 6)}$$

$$5 \leq x \leq 31 \quad \text{(Equation 3)}$$

$$50 \leq y \leq 75 \quad \text{(Equation 4)}$$

4. The oxygenator of hollow fiber membrane type according to claim 1, wherein the following relationship is satisfied between said thickness (x: mm) of said hollow fiber membrane bundle and said loading density (y: %) of said hollow fiber membranes to said annular space (z) formed between said outer surface and said inner surface of said hollow fiber membrane bundle:

$$y \leq -0.75x + 82 \quad \text{(Equation 5)}$$

$$y \geq -0.85x + 74 \quad \text{(Equation 6)}$$

$$5 \leq x \leq 31 \quad \text{(Equation 3)}$$

$$53 \leq y \leq 73 \quad \text{(Equation 7)}$$

5. The oxygenator of hollow fiber membrane type according to any one of claims 1 through 4, wherein said housing comprises an inner tubular member having a plurality of blood flowing apertures provided to a side wall thereof and an outer tubular member receiving said inner tubular member; said hollow fiber membrane bundle is located between said inner tubular member and said outer tubular member; and said housing has an interior of said inner tubular member communicating with one of said blood inlet or said blood outlet and an interior of said outer tubular member communicating with the other of said blood inlet or said blood outlet.

**6.** The oxygenator of hollow fiber membrane type according to any one of claims 1 through 5, wherein said hollow fiber membranes are wound around a peripheral surface of said inner tubular member having blood flowing apertures formed thereon.

**7.** The oxygenator of hollow fiber membrane type according to any one of claims 1 through 6, wherein said hollow fiber membrane bundle is formed by winding said hollow fiber membranes one by one or a combination of a plurality thereof around said inner tubular member in such a manner that said hollow fiber membranes are spaced at substantially regular intervals.

**8.** The oxygenator of hollow fiber membrane type according to any one of claims 1 through 7, wherein an interval between said hollow fiber membranes parallel with and adjacent to each other is set to 1/10 - 1/1 of an outer diameter of said hollow fiber membrane.

**9.** The oxygenator of hollow fiber membrane type according to any one of claims 1 through 8, wherein said hollow fiber membrane bundle is formed by winding said hollow fiber membranes one by one or a combination of a plurality thereof around said inner tubular member in such a manner that said hollow fiber membranes are spaced at substantially regular intervals; and when said hollow fiber membranes are wound around said inner tubular member , said hollow fiber membrane bundle is formed by operating a rotary member for rotating said inner tubular member and a winder for threading said hollow fiber membranes in such a manner that said rotary member and said winder satisfy the following equation:

traverse [mm/lot] x n (integer) = traverse swinging width x 2 ± (outer diameter of hollow fiber membrane + interval between adjacent hollow fiber membranes) x number of hollow fiber membranes wound on inner tubular member. (Equation 8)

FIG. 1

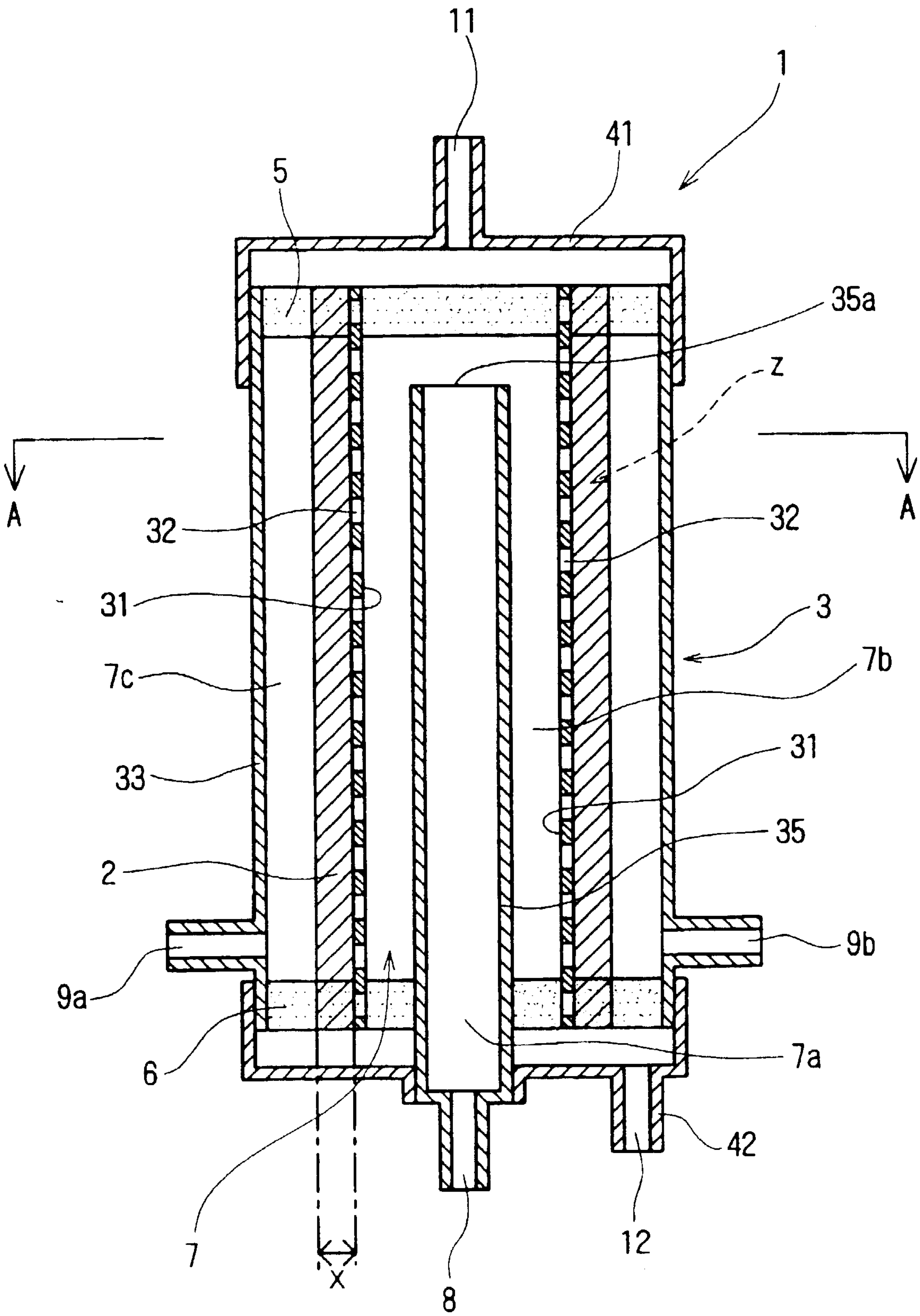

FIG. 2

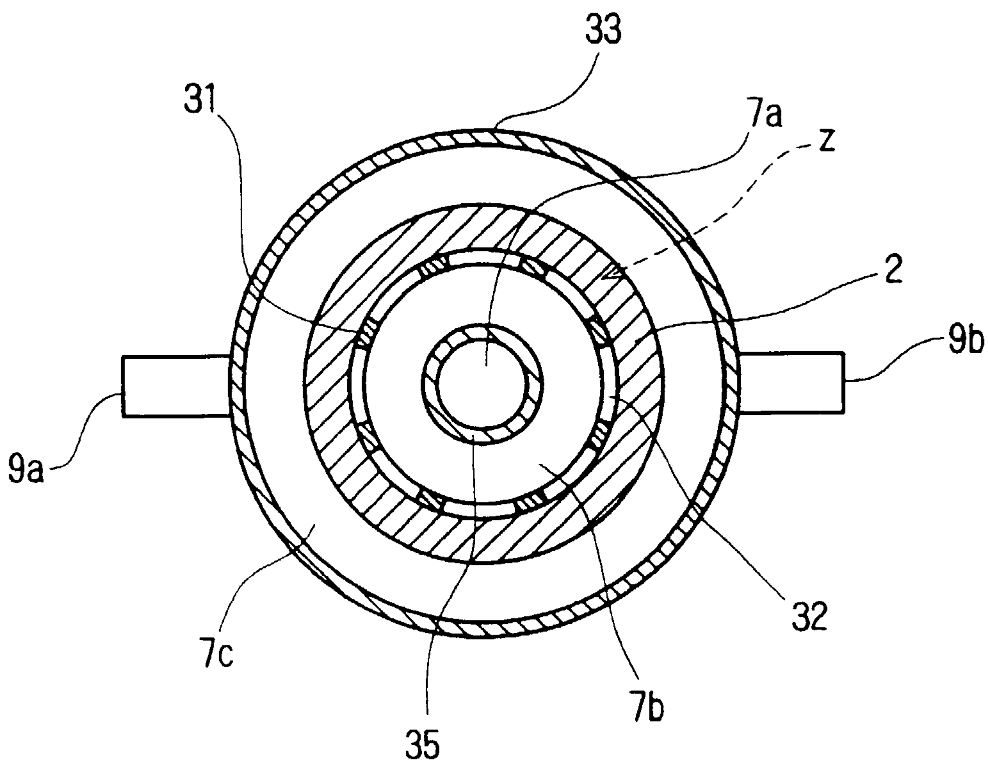

FIG. 3

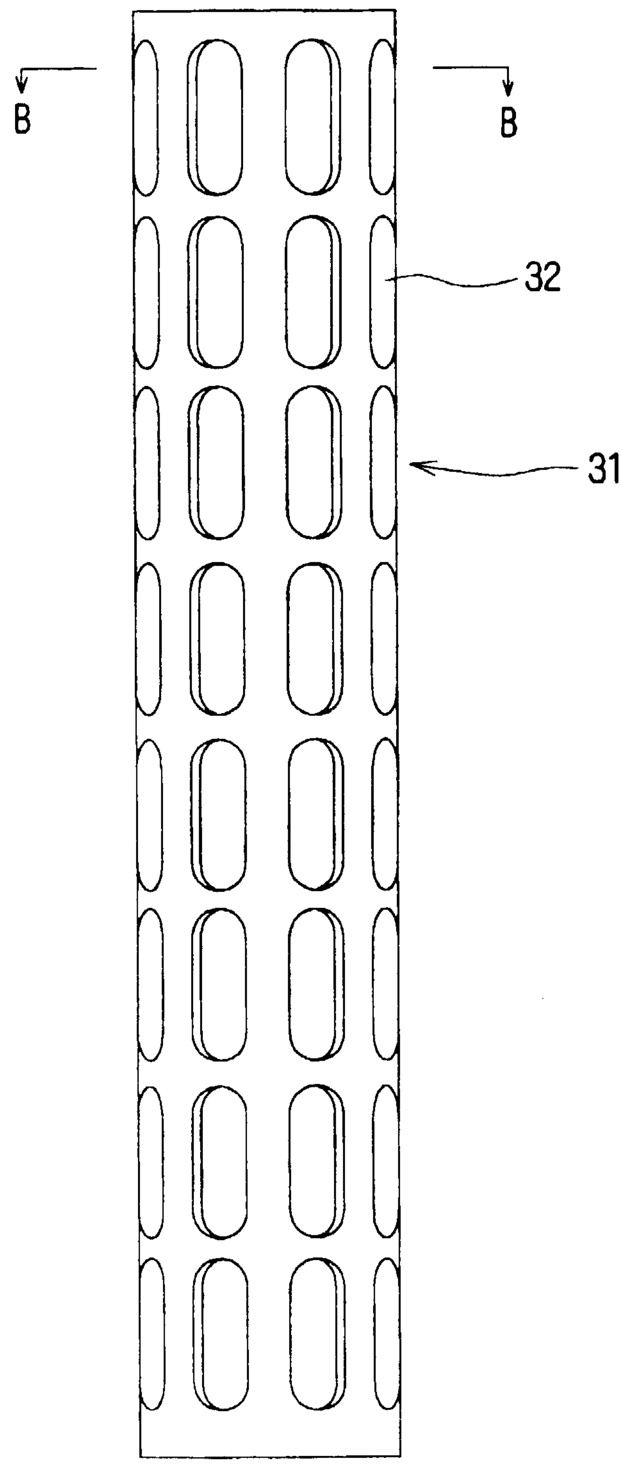

FIG. 4

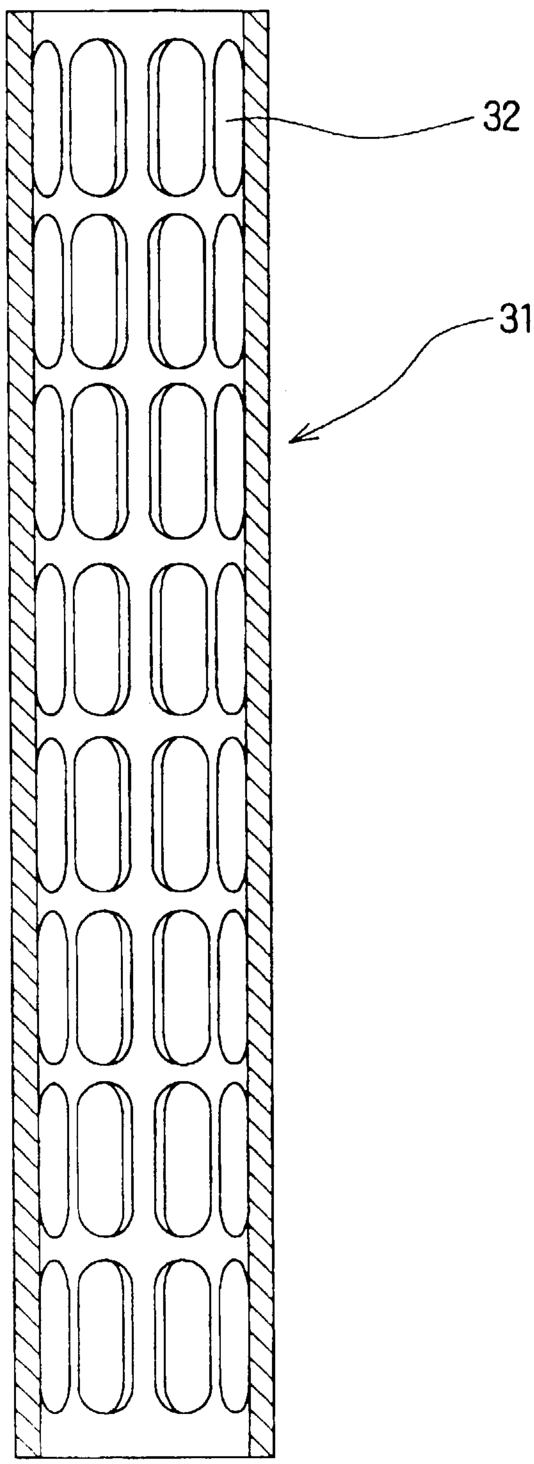

FIG. 5

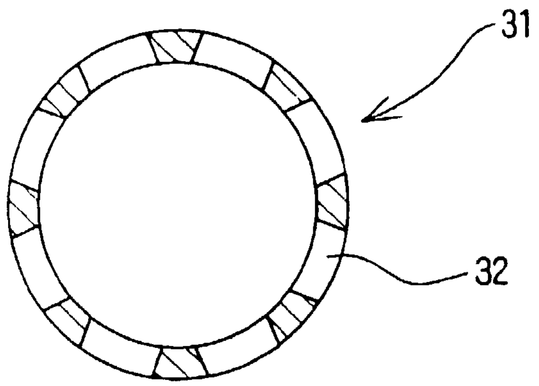

FIG. 6

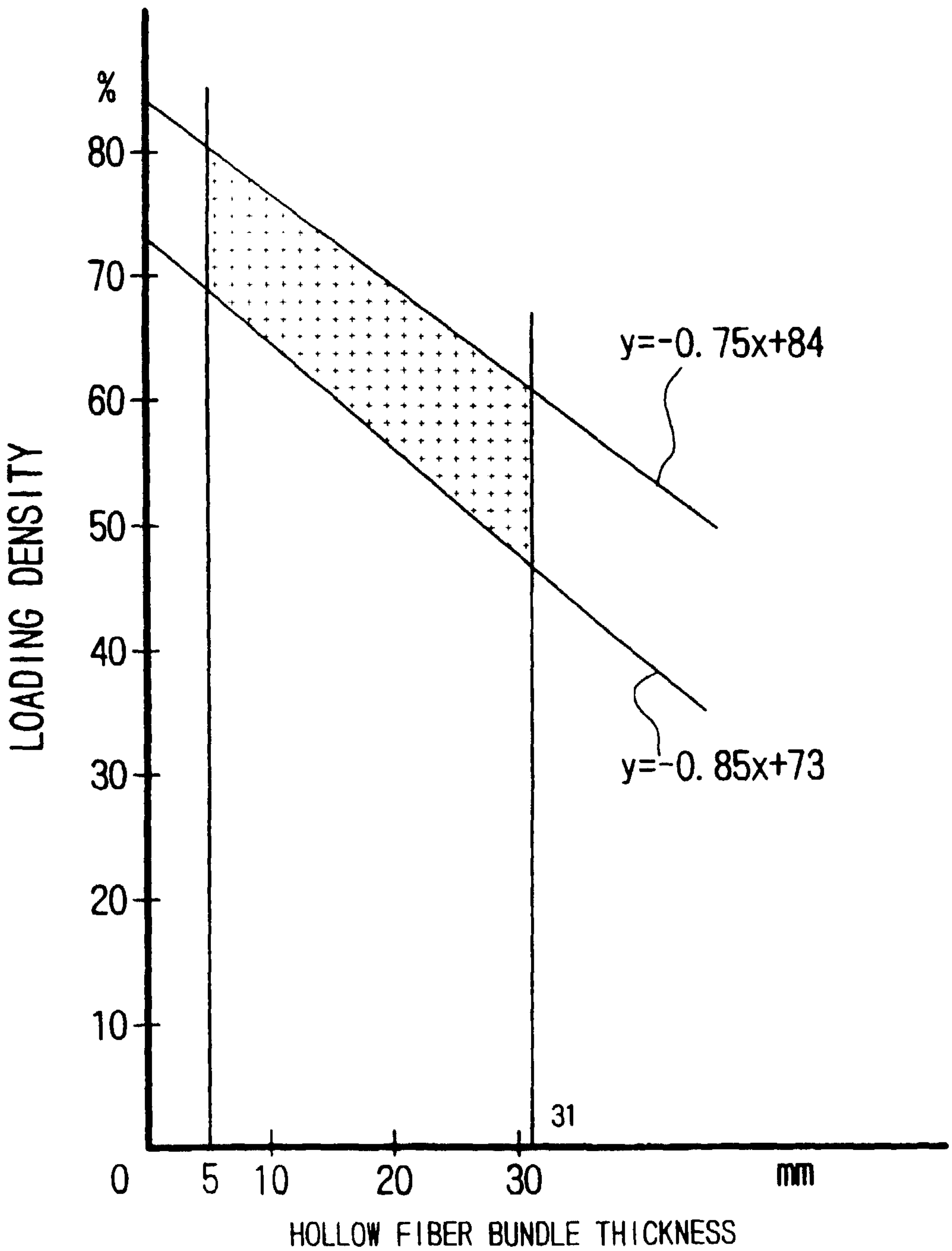
%
80
70
60
50
40
30
20
10
0
5
10
20
30
31
mm
LOADING DENSITY
HOLLOW FIBER BUNDLE THICKNESS
y=-0. 75x+84
y=-0. 85x+73

FIG. 7

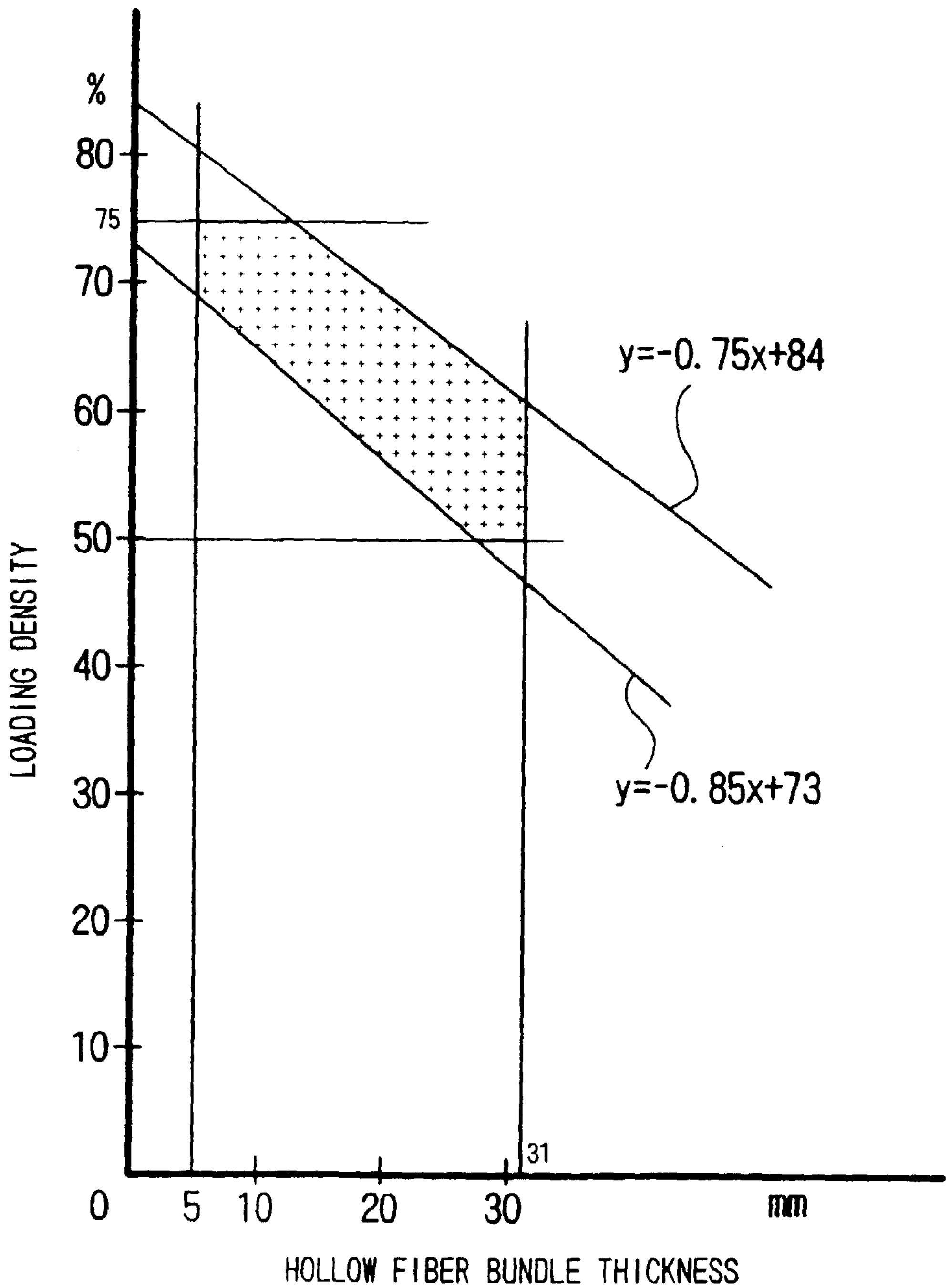
%
80
75
70
60
50
40
30
20
10
0
5
10
20
30
31
mm
LOADING DENSITY
HOLLOW FIBER BUNDLE THICKNESS
y=-0.75x+84
y=-0.85x+73

FIG. 8

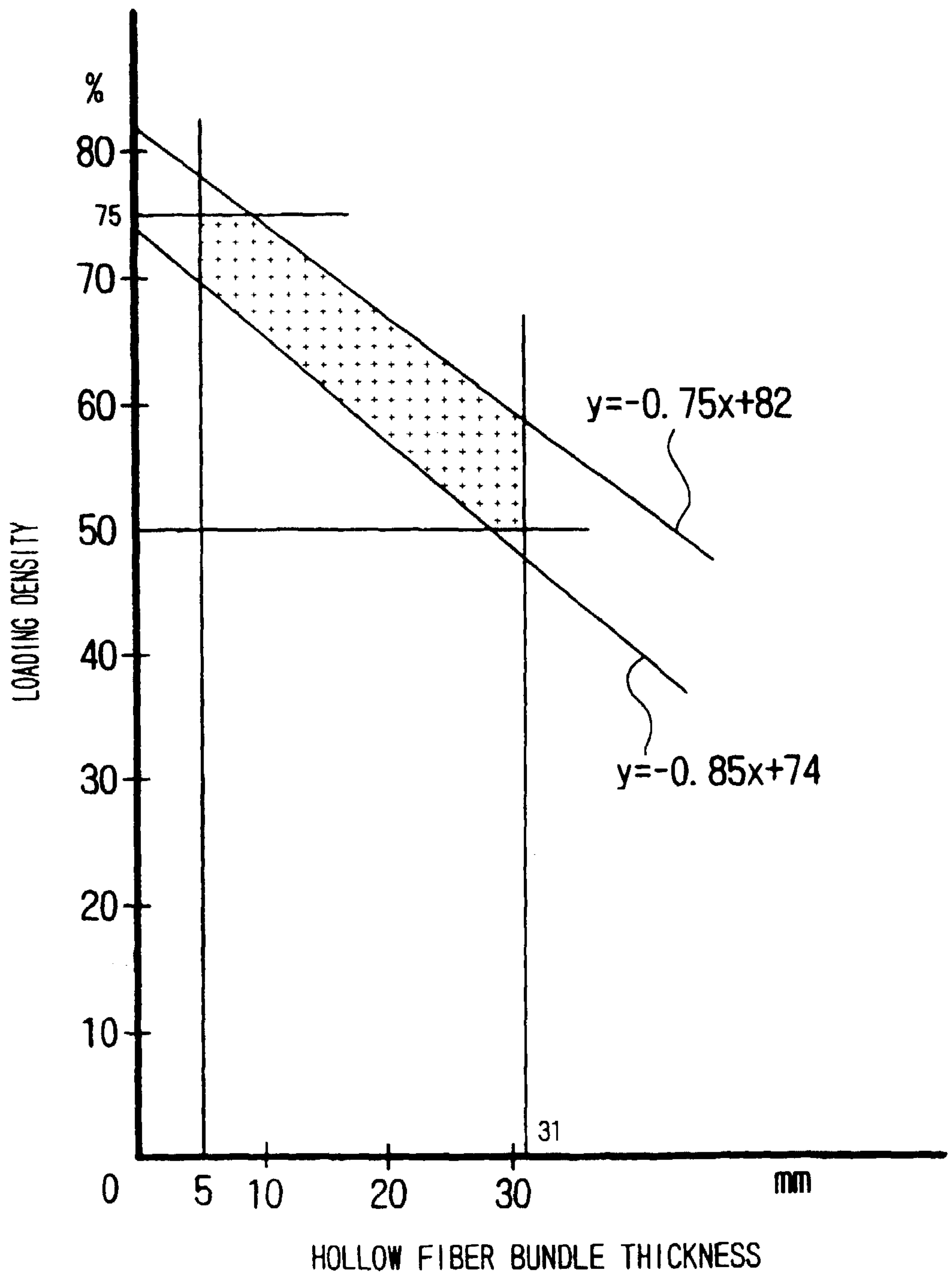
%
80
75
70
60
50
40
30
20
10
0
5
10
20
30
31
mm
y=-0.75x+82
y=-0.85x+74
LOADING DENSITY
HOLLOW FIBER BUNDLE THICKNESS

FIG. 9

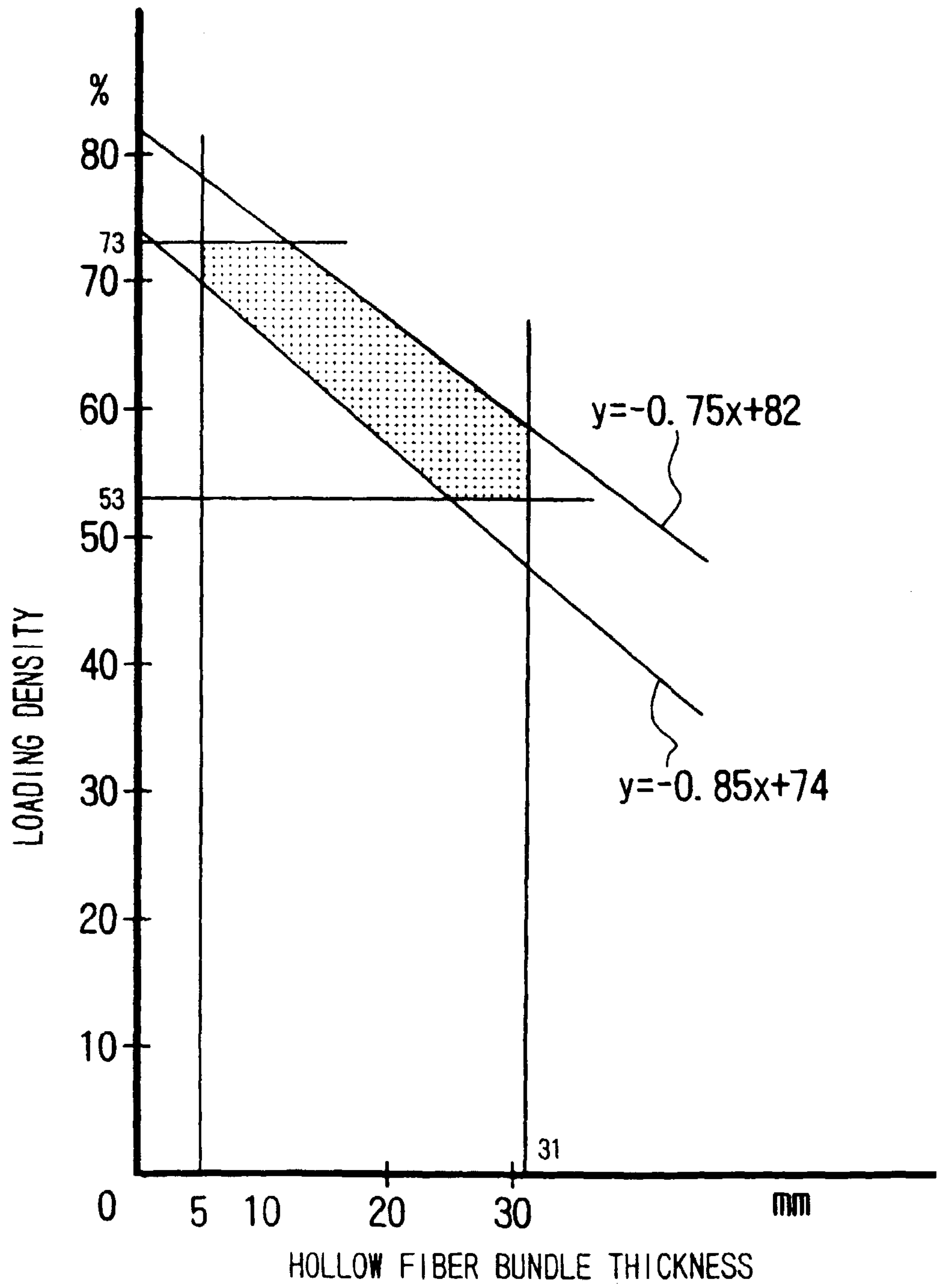
%
80
73
70
60
53
50
40
30
20
10
0
LOADING DENSITY
y=-0. 75x+82
y=-0. 85x+74
31
5
10
20
30
mm
HOLLOW FIBER BUNDLE THICKNESS

European Patent Office

# EUROPEAN SEARCH REPORT

Application Number

EP 98402005.7

| Category | Citation of document with indication, where appropriate, of relevant passages | Relevant to claim | CLASSIFICATION OF THE APPLICATION (Int. Cl. 6) |
|---|---|---|---|
| A | US 5346621 A (W. HAWORTH et al.) 13 September 1994 (13.09.94), totality, especially fig. 1,6, column 4, line 13 - column 6, line 13. -- | 1-9 | A 61 M 1/18 |
| A | US 5230862 A (G. BERRY et al.) 27 July 1993 (27.07.93), totality, especially fig. 1-4a, column 4, line 60 - column 8, line 53. -- | 1, 5-9 | |
| A | EP 0089122 A2 (CORDIS DOW CORP.) 21 September 1983 (21.09.83), fig. 1-9, page 8, line 16 - page 13, line 23. -- | 1, 5-9 | TECHNICAL FIELDS SEARCHED (Int. Cl.6) |
| A | US 4620965 A (H. FUKUSAWA et al.) 04 November 1986 (04.11.86), abstract, fig. 3, column 7, lines 9-51. -- | 1-4 | A 61 M<br>B 01 D |
| A | US 4239729 A (H. HASEGAWA et al.) 16 December 1980 (16.12.80), abstract, fig. 5, column 7, lines 4-33. -- | 1-4 | |
| A | US 5162101 A (L. COSENTINO et al.) 10 November 1992 (10.11.92), abstract, fig. 1,12,13, column 6, line 37 - column 7, line 24. ---- | 1-4 | |

The present search report has been drawn up for all claims

| Place of search | Date of completion of the search | Examiner |
|---|---|---|
| VIENNA | 23-11-1998 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

## ANNEX TO THE EUROPEAN SEARCH REPORT ON EUROPEAN PATENT APPLICATION NO. EP 98402005.7

This annex lists the patent family members relating to the patent documents cited in the above-mentioned search report.
The members are as contained in the EPIDOS INPADOC file on 25.11.1998.
The European Patent Office is in no way liable for these particulars which are merely given for the purpose of information.

| Patent document cited in search report | Publication date | Patent family member(s) | Publication date |
|---|---|---|---|
| US A 5346621 | 13-09-94 | AU A1 67877/94 | 12-12-94 |
| | | EP A1 652779 | 17-05-95 |
| | | EP A4 652779 | 27-03-96 |
| | | JP T2 7509171 | 12-10-95 |
| | | WO A1 9426327 | 24-11-94 |
| US A 5230862 | 27-07-93 | AU A1 25528/92 | 16-03-93 |
| | | EP A1 600035 | 08-06-94 |
| | | EP A4 600035 | 01-02-95 |
| | | JP T2 6509733 | 02-11-94 |
| | | WO A1 9303828 | 04-03-93 |
| EP A2 89122 | 21-09-83 | AT E 35621 | 15-07-88 |
| | | AU A1 11143/83 | 25-08-83 |
| | | AU B2 565652 | 24-09-87 |
| | | BR A 8300807 | 16-11-83 |
| | | CA A1 1190443 | 16-07-85 |
| | | DD A5 207656 | 14-03-84 |
| | | DE CO 3377331 | 18-08-88 |
| | | DK AO 712/83 | 14-02-83 |
| | | DK A 712/83 | 20-08-83 |
| | | DK B 160857 | 29-04-91 |
| | | DK C 160857 | 21-10-91 |
| | | EP A3 89122 | 25-04-84 |
| | | EP B1 89122 | 13-07-88 |
| | | JP A2 58155862 | 16-09-83 |
| | | JP B4 5020111 | 18-03-93 |
| | | NO A 830578 | 22-08-83 |
| | | NO B 156075 | 13-04-87 |
| | | NO C 156075 | 22-07-87 |
| | | NZ A 203283 | 08-11-85 |
| US A 4620965 | 04-11-86 | CA A1 1216207 | 06-01-87 |
| | | CA A2 1238831 | 05-07-88 |
| | | DE CO 3381625 | 12-07-90 |
| | | DE CO 3382435 | 21-11-91 |
| | | DE CO 3382483 | 30-01-92 |
| | | DE CO 3382591 | 20-08-92 |
| | | DE T2 3382591 | 11-03-93 |
| | | EP A2 103899 | 28-03-84 |
| | | EP A3 103899 | 25-07-84 |
| | | EP A1 240035 | 07-10-87 |
| | | EP A2 243796 | 04-11-87 |
| | | EP A3 243796 | 03-08-88 |
| | | EP A1 306613 | 15-03-89 |
| | | EP B1 103899 | 06-06-90 |
| | | EP B1 240035 | 16-10-91 |
| | | EP B1 306613 | 18-12-91 |
| | | EP B1 243796 | 15-07-92 |
| | | JP A2 59067963 | 17-04-84 |
| | | JP B4 62037994 | 14-08-87 |
| | | US E 33932 | 19-05-92 |
| | | JP A2 59057661 | 03-04-84 |
| | | JP B4 62037993 | 14-08-87 |
| | | JP A2 59055256 | 30-03-84 |
| | | JP B4 62037992 | 14-08-87 |
| US A 4239729 | 16-12-80 | AU A1 47826/79 | 13-12-79 |
| | | AU B2 527493 | 10-03-83 |
| | | BR A 7903583 | 22-01-80 |
| | | CA A1 1128393 | 27-07-82 |
| | | DE CO 2960682 | 19-11-81 |
| | | EP A1 5866 | 12-12-79 |
| | | EP B1 5866 | 26-08-81 |
| | | JP A2 54160098 | 18-12-79 |
| | | ZA A 7902810 | 25-06-80 |
| US A 5162101 | 10-11-92 | AU A1 37311/89 | 13-08-90 |
| | | CA AA 2006324 | 13-07-90 |
| | | ES AF 2014918 | 16-07-90 |
| | | WO A1 9007943 | 26-07-90 |
| | | CA C 2006324 | 14-02-95 |
| | | DE CO 68922908 | 06-07-95 |
| | | DE T2 68922908 | 02-11-95 |
| | | EP A1 453441 | 30-10-91 |
| | | EP A4 453441 | 05-02-92 |
| | | EP B1 453441 | 31-05-95 |

For more details about this annex see Official Journal of the European Patent Office, No. 12/82.